# EUROPÄISCHE PATENTSCHRIFT

(11) **EP 2 925 276 B1**
(45) Veröffentlichungstag und Bekanntmachung des Hinweises auf die Patenterteilung: **06.11.2019**
(21) Anmeldenummer: 13795494.7
(22) Anmeldetag: 25.11.2013
(51) Int. Cl.: A61K 6/027, C03C 10/00, C03C 4/00, A61K 6/02, B22D 13/06

(54) **VERFAHREN ZUM HERSTELLEN VON ZAHNERSATZ**
METHOD FOR PRODUCING A DENTAL PROSTHESIS
PROCÉDÉ DE FABRICATION DE PROTHÈSE DENTAIRE

(30) Priorität: 30.11.2012 DE 102012111683
(43) Veröffentlichungstag der Anmeldung: 07.10.2015
(73) Patentinhaber: DENTSPLY SIRONA Inc., York, PA 17401-2991 (US)
(72) Erfinder: KREUDER, Peter, 61231 Bad Nauheim (DE)
(74) Vertreter: Stoffregen, Hans-Herbert
(86) Internationale Anmeldenummer: PCT/EP2013/074634
(87) Internationale Veröffentlichungsnummer: WO 2014/082969

(56) Entgegenhaltungen:
- EP-A1- 0 225 279
- DE-A1-102010 050 275
- US-A- 4 431 420
- US-A- 5 507 981
- US-A- 5 925 180
- US-A1- 2012 248 642

## Beschreibung

Die Erfindung bezieht sich auf ein Verfahren zum Herstellen von Zahnersatz auf der Basis von Lithiumsilikat-Glas oder -Glaskeramik, umfassend zumindest die Verfahrensschritte
- Schmelzen eines Pulvergemisches, das zumindest SiO2, Li2O, Al2O3 enthält, und
- Gießen der so hergestellten Schmelze oder einer aus dem Pulver hergestellten Schmelze in eine von einer Einbettmasse umgebene dem herzustellenden Zahnersatz entsprechende Negativform.

Um Zahnersatz auf der Basis von Lithiumsilikat-Glaskeramik herzustellen, ist es bekannt, zylinderförmige Pellets herzustellen und diese sodann in einer Muffel zu verpressen (s. z. B. EP 1 484 031 B1). Dieses Verfahren hat sich bewährt und durchgesetzt, gleichwenn auch Vorschläge bekannt sind, nach denen eine Lithiumsilikat-Glasschmelze vergossen wird, wie dies der US 4 515 634 A zu entnehmen ist.

In der DE 29 49 619 A1 wird beschrieben, dass eine Schmelze auf Lithiumsilikat-Basis gegossen werden kann, um eine Zahnrestaurierung herzustellen. Das Glas enthält kein P₂O₅.

Zur Herstellung eines Vorprodukts auf Lithiumsilikat-Basis schlägt die US-A-5 698 482 vor, Pellets durch Gießen oder uniaxiales oder isostatisches Pressen herzustellen.

Aus der DE 10 2009 060 274 A1 sowie der WO 2012/059143 A1 sind Verfahren zu entnehmen, um aus Lithiumdisilikat-Glaskeramik einen Zahnersatz herzustellen.

Der US 5 507 981 A ist ein Verfahren zur Herstellung eines Zahnersatzes zu entnehmen, bei dem eine Keramikschmelze einer Negativform durch Vakuum oder Druck zugeführt wird. Ein Verfahren zur Herstellung von Lithiumsilikat-Gläsem oder -Glaskeramiken ist aus der DE 10 2010 050 275 A1 bekannt.

Der vorliegenden Erfindung liegt die Aufgabe zu Grunde, ein Verfahren der eingangs genannten Art so weiterzubilden, dass reproduzierbar Zahnersatz durch Gießen auf der Basis von Lithiumsilikat-Glas bzw. -Glaskeramik hergestellt werden kann. Dabei sollen im Vergleich zum Stand der Technik herstellungstechnische Vereinfachungen gegeben sein. Auch soll ein Ausgangsmaterial zur Verfügung gestellt werden, das problemlos handhabbar ist.

Zur Lösung der Aufgabe ist u. a. vorgesehen,
- Schmelzen eines Pulvers mit der Zusammensetzung in Gew.-%

| | | | |
|---|---|---|---|
| SiO2 | 50 | - | 70 % |
| Li2O | 5 | - | 25 % |
| Al2O3 | 0.1 | - | 20 % |
| K2O | 0.1 | - | 15 % |
| CeO2 | 0.1 | - | 15 % |
| B2O3 | 0 | - | 5 % |
| P2O5 | 0 | - | 15 % |
| Tb2O3 | 0 | - | 2 % |
| ZrO2 | 0 | - | 15 % |
| ZnO2 | 0 | - | 4 % |

sowie zumindest einem Additiv zwischen 0,1 und 5 % eines Oxids aus der Gruppe BaO, CaO, MgO, MnO, Er2O3, Gd2O3, Na2O, Pr2O3, Pr6O11, Sm2O3, TiO2, V2O5, Y2O3,
- Herstellen von aus der Schmelze erstarrten kugel- oder linsenförmigen Glaspartikeln,
- Portionieren der Glaspartikel und Einfüllen in einen Gießtiegel,
- Schmelzen der Glaspartikel in dem Gießtiegel und Einstellen auf eine Viskosität ν mit 4 dPa·s ≤ ν ≤ 80 dPa·s,
- Gießen der so hergestellten Schmelze in eine dem Zahnersatz entsprechende von einer Einbettmasse umgebene Negativform,
- Erstarrenlassen der Schmelze in der Negativform,
- Kristallisieren von Lithiummetasilikat als Hauptkristallphase aus der erstarrten Schmelze durch Anwendung einer ersten Wärmebehandlung bei einer Temperatur zwischen 600 °C und 760 °C über eine Zeit zwischen 20 min und 120 min,
- Kristallisieren von Lithiumdisilikat als Hauptkristallphase in einer zweiten Wärmebehandlung bei einer Temperatur zwischen 760 °C und 860 °C über eine Zeit zwischen 5 min und 60 min
- wobei die erste und zweite Wärmebehandlung in der Einbettmasse oder nach Entfernen der erstarrten Schmelze aus der Einbettmasse erfolgt.

Insbesondere sieht die Erfindung vor, dass der Zahnersatz durch Schleuder- oder Vakuumdruckgießen hergestellt wird.

Bevorzugterweise zeichnet sich die Erfindung dadurch aus, dass die Schmelze mit einer Viskosität v mit 9 dPa·s bis 40 dPa·s gegossen wird.

Zur Lösung der Aufgabe sieht die Erfindung im Wesentlichen auch vor, dass der Zahnersatz aus der Schmelze mit der Zusammensetzung in Gew.-%:

| | | | |
|---|---|---|---|
| SiO2 | 50 | - | 70 % |
| Li2O | 5 | - | 25 % |
| Al2O3 | 0.1 | - | 20 % |
| K2O | 0.1 | - | 15 % |
| CeO2 | 0.1 | - | 15 % |
| B2O3 | 0 | - | 5 % |
| P2O5 | 0 | - | 15 % |
| Tb2O3 | 0 | - | 2 % |
| ZrO2 | 0 | - | 15 % |
| ZnO2 | 0 | - | 4 % |

sowie zumindest ein Additiv zwischen 0,1 % und 5 %, aus der Gruppe BaO, CaO, MgO, MnO, Er2O3, Gd2O3, Na2O, Pr2O3, Pr6O11, Sm2O3, TiO2, V205, Y2O3 im Schleudergieß- oder Vakuumdruckgießverfahren hergestellt wird.

Insbesondere ist vorgesehen, dass die Zusammensetzung zusätzlich zumindest ein Oxid eines Übergangsmetalls mit einer Ordnungszahl zwischen 41 und 79 wie La, Nb und Ta mit einem Gew.-%-Anteil zwischen 0 und 8 % enthält. Der Gew.-%-Anteil von einem Oxid der Übergangsmetalle mit der Ordnungszahl 59, 62, 64, 68 beträgt maximal 5 %.

Bevorzugterweise wird eine Zusammensetzung zur Herstellung von Zahnersatz verwendet in Gew.-% mit:

| | | | |
|---|---|---|---|
| SiO2 | 55 | - | 65 % |
| Li2O | 10 | - | 20 % |
| Al2O3 | 0,2 | - | 15 % |
| K2O | 0,2 | - | 10 % |
| CeO2 | 0,1 | - | 10 % |
| B2O3 | 0 | - | 5 % |
| P2O5 | 1 | - | 10 % |
| Tb2O3 | 0 | - | 2 % |
| ZrO2 | 2 | - | 15 % |
| ZnO2 | 0 | - | 2 % |

sowie zumindest ein Additiv zwischen 0,1 % und 5 % aus der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung zusätzlich zumindest ein Oxid eines Übergangsmetalls mit einer Ordnungszahl zwischen 41 und 79 wie La, Nb und Ta mit einem Gew.-%-Anteil zwischen 0 % und 8 % enthält. Der Gew.-%-Anteil von einem Oxid der Übergangsmetalle mit der Ordnungszahl 59, 62, 64, 68 beträgt maximal 5 %.

Noch bevorzugter wird eine Zusammensetzung zur Herstellung des Zahnersatzes verwendet in Gew.-% mit:

| | | | |
|---|---|---|---|
| SiO2 | 57 | - | 63 % |
| Li2O | 11 | - | 19 % |
| Al2O3 | 0,5 | - | 10 % |
| K2O | 0,5 | - | 10 % |
| CeO2 | 0,1 | - | 10 % |
| B2O3 | 0 | - | 5 % |
| P2O5 | 1 | - | 10 % |
| Tb2O3 | 0 | - | 2 % |
| ZrO2 | 4 | - | 15 % |
| ZnO2 | 0 | - | 2 % |

sowie zumindest einem Additiv zwischen 0,1 % und 5 % bestehend aus Oxiden der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung außerdem zumindest ein Oxid eines Übergangsmetalls mit einer Ordnungszahl zwischen 41 und 79 wie La, Nb und Ta mit einem Gew.-%-Anteil zwischen 0 und 6 % enthält. Der Gew.-%-Anteil von einem Oxid der Übergangsmetalle mit der Ordnungszahl 59, 62, 64, 68 beträgt maximal 5 %.

Besonders bevorzugt wird eine Zusammensetzung zur Herstellung des Zahnersatzes verwendet in Gew.-% mit:

| | | | |
|---|---|---|---|
| SiO2 | 58 | - | 62 % |
| Li2O | 13 | - | 19 % |
| Al2O3 | 1 | - | 6 % |
| K2O | 1 | - | 5 % |
| CeO2 | 0,1 | - | 5 % |
| B2O3 | 0 | - | 4 % |
| P2O5 | 2 | - | 8 % |
| Tb2O3 | 0 | - | 2 % |
| ZrO2 | 8 | - | 12 % |
| ZnO2 | 0 | - | 1 % |

sowie zumindest einem Additiv zwischen 0,1 % und 4 % aus der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung außerdem zumindest ein Oxid eines Übergangsmetalls mit einer Ordnungszahl zwischen 41 und 79 wie La, Nb und Ta mit einem Gew.-%-Anteil zwischen 0 % und 5 % enthält. Der Gew.-%-Anteil von einem Oxid der Übergangsmetalle mit der Ordnungszahl 59, 62, 64, 68 beträgt maximal 4 %.

Eigenerfinderisch ist, dass die aus dem Pulvergemisch hergestellte Schmelze vertropft wird, dass die so hergestellten Tropfen eine Kühlstrecke durchfallen, in der zumindest eine oberflächliche Verfestigung der Tropfen erfolgt, und dass nach Durchfallen der Kühlstrecke und ggfs. einem Abschrecken der Tropfen diese einer Aufnahme zugeführt werden, die vorzugsweise eine Flüssigkeit wie Wasser oder eine Hochtemperaturwatte enthält.

Durch diese Maßnahmen erhält man gleichförmige kugel- oder linsenförmig verfestigte Endtropfen, die sodann vor dem Gießen aufgeschmolzen werden.

Somit steht ein Ausgangsprodukt für das Gießen zur Verfügung, das problemlos handhabbar ist. Ein Befüllen eines Schmelztiegels für das zahntechnische Vergießen ist, je nach Masse bzw. Größe des zu gießenden Objekts (z. B. Zahnkrone, Zahnbrücke, Abutment etc.), ohne Schwierigkeit im gewünschten Umfang möglich.

Um das Pulvergemisch aufzuschmelzen, ist vorgesehen, dass das Pulvergemisch über eine Zeit t1 mit 4 h ≤ t1 ≤ 12 h einer Temperatur T1 mit 1500 °C ≤ T1 ≤ 1600 °C ausgesetzt wird. Das Schmelzen erfolgt in einem geeigneten Schmelztiegel aus Pt-Legierungen z. B. Pt/Rh 80/20, in dem auch ein Entgasen erfolgt. Die so hergestellte Schmelze, also das Glas, wird sodann einer separat beheizbaren Düse zugeführt. Die Temperatur T2 der Düse sollte im Bereich zwischen 1250 °C und 1450 °C liegen. Das heiße flüssige Glas wird sodann tropfenweise aus der Düse abgegeben. Hierzu kann die Düse in Schwingung versetzt werden. Unabhängig hiervon sollte die Temperatur T2 mit 1250 °C ≤ T2 ≤ 1450 °C eingestellt werden.

Die aus der Düse austretenden Tropfen durchfallen sodann eine Kühlstrecke, in der zumindest eine oberflächliche Verfestigung erfolgt. Diese wird benötigt, da die Tropfen bevorzugterweise sodann auf ein Blechelement wie Metallblech auftreffen, das eine Temperatur zwischen Raumtemperatur und 500 °C aufweisen kann. Durch das Auftreffen der Tropfen auf das Metallelement erfolgt eine Abplattung der Tropfen und eine Umlenkung dieser in eine Aufnahme, die vorzugweise mit Hochtemperaturwatte ausgelegt ist oder aus einem mit Wasser gefüllten Behälter besteht.

Die Tropfen können von dem Metallelement über z. B. eine Schräge der Hochtemperaturwatte bzw. dem Wasser zugeführt werden.

Die Form der ausgehärteten Tropfen ist mit einer auf der Unterseite abgeflachten Kugel bzw. als "Halbkugel" oder "Linse" zu umschreiben. Die Größe der Tropfen liegt bei 2 mm bis 9 mm am äquatorialen Durchmesser mit einer Streuung von +/- 0,1 mm bis +/-1mm. Die Höhe der "Halbkugel" liegt zwischen 1 mm und 5 mm.

Um etwaige innere Spannungen, die bei der Erstarrung bzw. Abschreckung entstanden sein können, abzubauen, können die Tropfen in einem Entspannungsofen von einer Temperatur T3 mit 350 °C ≤ T3 ≤ 500 °C, insbesondere T3 in etwa 450 °C, stufenweise über einen Zeitraum t3 mit 2 h ≤ t3 ≤ 6 h auf Raumtemperatur abgekühlt werden.

Bei der zahntechnischen Weiterverarbeitung von gegossenem Zahnersatz wird vorgeschlagen, zur Erzielung der erforderlichen Festigkeit Kristallphasen in dem Glas auszuscheiden. Hierzu wird vorgeschlagen, dass die in der Negativform erstarrte Schmelze, also das Glas in der Negativform, einer Wärmebehandlung ausgesetzt wird. So kann z. B. in einem ersten Wärmebehandlungsschritt bei einer Temperatur T4 mit 600°C ≤ T4 ≤ 760 °C über eine Zeit t4 mit 20 min ≤ t4 ≤ 2 h das Lithiummetasilikat ausgeschieden werden.

Des Weiteren zeichnet sich die Erfindung alternativ dadurch aus, dass nach Erstarren der Schmelze der Guss aus der Einbettmasse entfernt und sodann der Guss einer ersten Wärmebehandlung über eine Zeit t5 mit 20 min ≤ t5 ≤ 120 min bei einer Temperatur T5 mit 600 °C ≤ T5 ≤ 760 °C unterzogen wird, bei der als Hauptkristallphase Lithiummetasilikat entsteht.

In einem zweiten Wärmebehandlungsschritt, der gleichfalls innerhalb der Negativform oder nach dem Entformen aus dieser erfolgen kann und bei dem als Hauptkristallphase Lithiumdisilikat ausgeschieden wird, kann der Guss einer Temperaturbehandlung über eine Zeit t6 mit 5 min ≤ t6 ≤ 60 min einer Temperatur T6 mit 760°C ≤ T6 ≤ 860 °C ausgesetzt werden.

Der Anteil der Kristallphase des Lithiumdisilikats beträgt nach entsprechenden Wärmebehandlungsschritten 20 Vol.-% bis 90 Vol.-% der Glaskeramik.

Sodann wird die Lithiumdisilikatglas-Keramik über eine Zeit t7 mit 10 min ≤ t7 ≤ 180 min auf Raumtemperatur abgekühlt.

Insbesondere ist vorgesehen, dass als Einbettmasse, in der die Negativform wie bei einem Muffelsystem in bekannter Weise ausgebildet wird, eine gipsgebundene Einbettmasse verwendet wird. Es kann aber auch eine Einbettmasse auf phosphatgebundener Basis verwendet werden.

Der Vorteil von gipsgebundener Einbettmasse ist, dass die Form problemlos ausgebettet werden kann, da die Einbettmasse z. B. in Wasser auflösbar ist.

Der Gießtiegel sollte ein Keramiktiegel oder Graphittiegel sein. Letzterer sollte innenseitig z. B. mit Bornitrid beschichtet (z. B. als Engobe) sein, um zu verhindern, dass Kohlenstoff bzw. andere Tiegelverunreinigungen in die Schmelze gelangt.

Zum Schmelzen der Tropfen wird der Schmelztiegel auf eine Temperatur T8 mit 1200 °C ≤ T8 ≤ 1300 °C erwärmt, wobei die Einbettmasse im Bereich der Negativform beim Gießen auf eine Temperatur T8 mit 600 °C ≤ T8 ≤ 800 °C eingestellt werden sollte. Bei Temperaturen größer als 710 °C müssen dafür phosphatgebundene Einbettmassen genommen werden.

Unabhängig hiervon sollte die Schmelze zum Gießen auf eine Viskosität ν mit 4 dPa·s ≤ ν ≤ 80 dPa·s, vorzugsweise 9 dPa·s ≤ ν ≤ 40 dPa·s, in dem Schmelztiegel eingestellt werden.

Weitere Einzelheiten, Vorteile und Merkmale der Erfindung ergeben sich nicht nur aus den Ansprüchen, den diesen zu entnehmen Merkmalen -für sich und/oder in Kombination-, sondern auch aus der nachfolgenden Beschreibung von bevorzugten Ausführungsbeispielen.

Es zeigen:
- Fig. 1: ein Flussdiagramm,
- Fig. 2: eine Prinzipdarstellung zur Herstellung von Tropfen und
- Fig. 3: eine Prinzipdarstellung eines Tropfens.

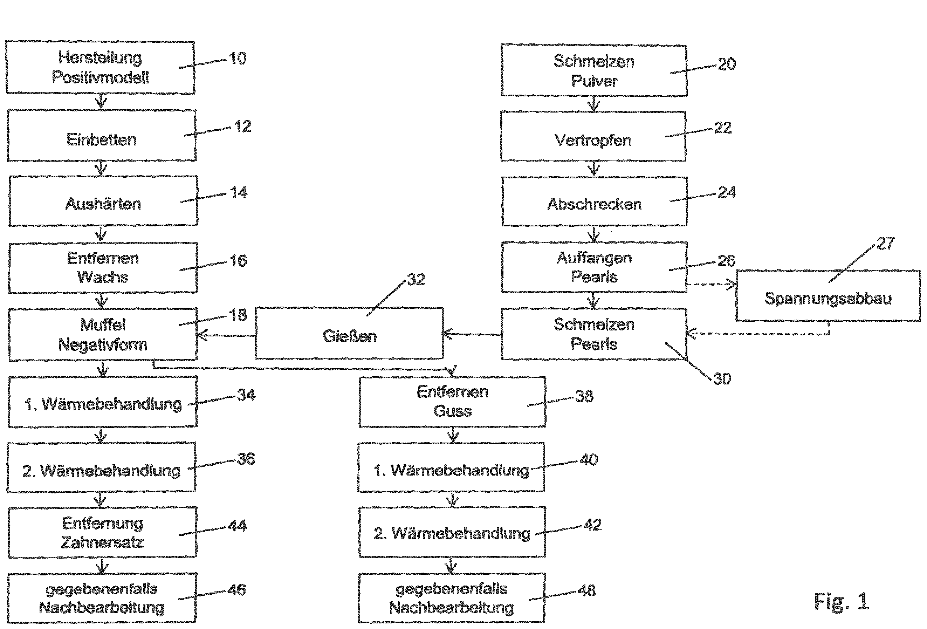

Um einen Zahnersatz durch Gießen herzustellen, sind nachstehend beschriebene Hauptverfahrensschritte durchzuführen, wie diese prinzipiell dem Flussdiagramm gemäß Fig. 1 zu entnehmen sind.

Zunächst wird eine Positivform des herzustellenden Zahnersatzes z. B. aus Wachs oder Kunststoff durch Modellieren von Hand oder z. B. im Rapid-Prototyping-Verfahren hergestellt (Verfahrensschritt 10). Das so hergestellte Positivmodell wird wie bei üblichen Muffelsystemen in eine Einbettmasse eingebettet, die insbesondere gipsgebunden ist. Sodann wird die Einbettmasse ausgehärtet und anschließend durch Erhitzen das Positivmodell entfernt (Verfahrensschritt 16), so dass eine ausgehärtete Einbettmasse, also eine Muffel mit in dieser vorhandenen Negativform zur Verfügung steht (Verfahrensschritt 18). Unabhängig hiervon wird zur Herstellung eines Lithiumsilikat-Glases ein Pulver aufgeschmolzen (Verfahrensschritt 20), das nachfolgende bevorzugte Zusammensetzung aufweisen kann:

| | | | |
|---|---|---|---|
| SiO2 | 50 | - | 70 % |
| Li2O | 5 | - | 25 % |
| Al2O3 | 0.1 | - | 20 % |
| K2O | 0.1 | - | 15 % |
| CeO2 | 0.1 | - | 15 % |
| B2O3 | 0 | - | 5 % |
| P2O5 | 0 | - | 15 % |
| Tb2O3 | 0 | - | 2 % |
| ZrO2 | 0 | - | 15 % |
| ZnO2 | 0 | - | 4 % |

sowie zumindest einem Additiv zwischen 0,1 und 5 % bestehend aus Oxiden der Gruppe BaO, CaO, MgO, MnO, Er2O3, Gd2O3, Na2O, Pr2O3, Pr6O11, Sm2O3, TiO2, V2O5, Y2O3.

Insbesondere ist vorgesehen, dass die Zusammensetzung zusätzlich zumindest ein Oxid eines Übergangsmetalls mit einer Ordnungszahl zwischen 41 und 79 wie La, Nb und Ta mit einem Gew.-%-Anteil zwischen 0 und 8 % enthält. Der Gew.-%-Anteil von einem Oxid der Übergangsmetalle mit der Ordnungszahl 59, 62, 64, 68 beträgt maximal 5 %.

Insbesondere wird eine Zusammensetzung benutzt, die besteht aus:

| | | | |
|---|---|---|---|
| SiO2 | 55 | - | 65 % |
| Li2O | 10 | - | 20 % |
| Al2O3 | 0.2 | - | 15 % |
| K2O | 0.2 | - | 10 % |
| CeO2 | 0.1 | - | 10 % |
| B2O3 | 0 | - | 5 % |
| P2O5 | 1 | - | 10 % |
| Tb2O3 | 0 | - | 2 % |
| ZrO2 | 2 | - | 15 % |
| ZnO2 | 0 | - | 2 % |

sowie zumindest einem Additiv zwischen 0,1 und 5 % bestehend aus Oxiden der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung außerdem zumindest ein Oxid eines Übergangsmetalls mit einer Ordnungszahl zwischen 41 und 79 wie La, Nb und Ta mit einem Gew.-%-Anteil zwischen 0 und 8 % enthält. Der Gew.-%-Anteil von einem Oxid der Übergangsmetalle mit der Ordnungszahl 59, 62, 64, 68 beträgt maximal 5 %.

Bevorzugterweise ist die Zusammensetzung wie folgt zu wählen:

| | | | |
|---|---|---|---|
| SiO2 | 57 | - | 63 % |
| Li2O | 11 | - | 19 % |
| Al2O3 | 0.5 | - | 10 % |
| K2O | 0.5 | - | 10 % |
| CeO2 | 0.1 | - | 10 % |
| B2O3 | 0 | - | 5 % |
| P2O5 | 1 | - | 10 % |
| Tb2O3 | 0 | - | 2 % |
| ZrO2 | 4 | - | 15 % |
| ZnO2 | 0 | - | 2 % |

sowie zumindest einem Additiv zwischen 0,1 und 5 % bestehend aus Oxiden der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung zusätzlich zumindest ein Oxid eines Übergangsmetalls mit einer Ordnungszahl zwischen 41 und 79 wie La, Nb und Ta mit einem Gew.-%-Anteil zwischen 0 und 6 % enthält. Der Gew.-%-Anteil von einem Oxid der Übergangsmetalle mit der Ordnungszahl 59, 62, 64, 68 beträgt maximal 5 %.

Besonders bevorzugterweise ist die Zusammensetzung wie folgt zu wählen:

| | | | |
|---|---|---|---|
| SiO2 | 58 | - | 62 % |
| Li2O | 13 | - | 19 % |
| Al2O3 | 1 | - | 6 % |
| K2O | 1 | - | 5 % |
| CeO2 | 0.1 | - | 5 % |
| B2O3 | 0 | - | 5 % |
| P2O5 | 2 | - | 8 % |
| Tb2O3 | 0 | - | 2 % |
| ZrO2 | 8 | - | 12 % |
| ZnO2 | 0 | - | 1 % |

sowie zumindest einem Additiv zwischen 0,1 und 4 % bestehend aus Oxiden der Gruppe BaO, CaO, MgO, MnO, Er2O3, Gd2O3, Na2O, Pr2O3, Pr6O11, Sm2O3, TiO2, V2O5, und Y2O3, wobei insbesondere vorgesehen ist, dass die Zusammensetzung außerdem zumindest ein Oxid eines Übergangsmetalls mit einer Ordnungszahl zwischen 41 und 79 wie La, Nb und Ta mit einem Gew.-%-Anteil zwischen 0 und 5 % enthält. Der Gew.-%-Anteil von einem Oxid der Übergangsmetalle mit der Ordnungszahl 59, 62, 64, 68 beträgt maximal 4 %.

Komponenten der Additiva können auch glasfärbende Oxide sein.

Unabhängig von der Art der Zusammensetzung ist anzumerken, dass die Gesamtgewichtsprozente der Komponenten der Pulvermischung in jeder Zusammensetzung mit den angegebenen Komponenten 100 Gewichtsprozente betragen.

Das Pulvergemisch wird in einem Schmelztiegel, der insbesondere aus hochtemperaturfesten Platinlegierungen wie z. B. Pt/Rh 80/20 oder Pt/Rh 90/10 dispersionsverstärkt besteht, über eine Zeitdauer t1 zwischen 4 h und 12 h bei einer Temperatur T1 zwischen 1500 °C und 1600 °C, insbesondere über einen Zeitraum von 4 h bei einer Temperatur von 1540 °C geschmolzen und geläutert. Das so hergestellte Glas wird sodann bevorzugterweise vertropft (Verfahrensschritt 22). Hierzu wird das Glas einer vorzugsweise in Schwingung versetzten Düse zugeführt, die eine Temperatur T2 zwischen 1250 °C und 1450 °C, insbesondere 1310 °C aufweist. Das Glas verlässt die Düse in Tropfenform. Beim Vertropfen der Schmelze zu den Halbkugeln ist eine Viskosität der Schmelze bevorzugt von 3 dPa·s bis 32 dPa·s einzustellen. Der Düsendurchmesser beträgt 2,0 mm bis 3,0 mm, vorzugsweise 2,6 mm, die Düsenlänge vorzugsweise 10 mm bis 40 mm und die Schwingfrequenz der Düse zwischen 40 Hz und 60 Hz, vorzugsweise 50 Hz.

Die Tropfen werden während des Durchfallens einer Kühlstrecke zumindest oberflächlich vorverfestigt, um sodann auf ein Metallelement wie Blech zu fallen, das eine Temperatur zwischen Raumtemperatur bis 500 °C, insbesondere im Bereich von Raumtemperatur bis 100 °C aufweisen sollte. Hierdurch erfolgt ein Abschrecken der Tropfen (Schritt 24). Die abgeschreckten Tropfen werden anschließend einem Behälter zugeführt (Schritt 26), der z. B. Hochtemperaturwatte oder Wasser enthalten kann. Es entstehen sogenannte Pearls, d. h., kugel- oder linsenförmige Tropfen, die aufgrund des durchgeführten Verfahrens weitgehend gleiche Abmessungen aufweisen. In bevorzugter Weise werden die Düsenstrecke, Temperatur des Metallelementes, Düsentemperatur und Temperatur des heißen Glases derart aufeinander abgestimmt, dass sich folgende Abmessungen für die Pearls ergeben: Hauptdurchmesser R mit 2 mm ≤ R ≤ 9 mm und Höhe H mit 1 mm ≤ H ≤ 5 mm.

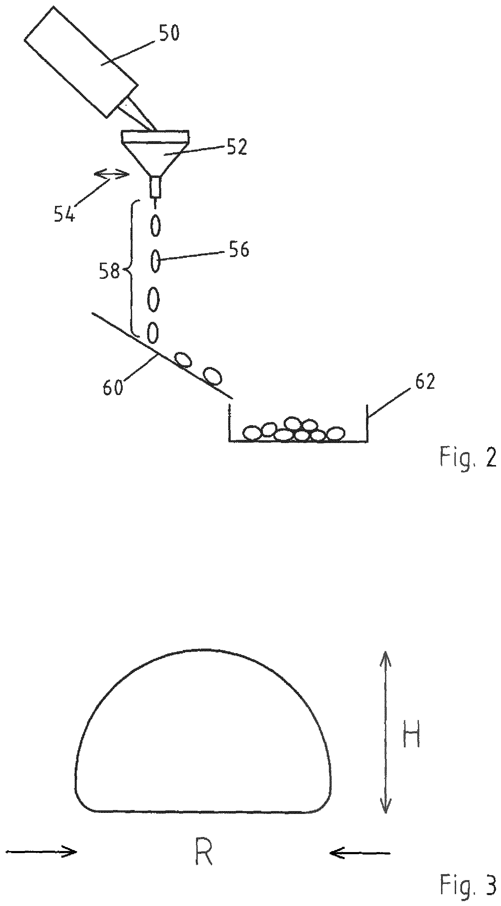

Einen Querschnitt eines entsprechenden Pearls oder Tropfens ist der Fig. 3 zu entnehmen. Der Querschnitt verdeutlicht, dass man an und für sich von einer Brötchenform mit glatter Oberfläche sprechen kann.

Die Pearls werden sodann geschmolzen (Schritt 30). Hierzu wird insbesondere ein Schmelztiegel aus Graphit oder Keramik benutzt. Bei der Verwendung eines Graphittiegels sollte dieser beschichtet sein. Hierzu kann Bornitrid benutzt werden (oder ein Tiegel vollständig aus Siliziumcarbid bzw. Bornitrid). Durch diese Maßnahme ist sichergestellt, dass Kohlenstoff in die Schmelze nicht eindringen kann. Der Schmelztiegel hat bevorzugterweise eine geschlossene Hohlzylinderform, in deren einer Stirnseite mittig eine Öffnung mit einem Durchmesser von vorzugsweise 8 mm vorhanden ist.

Sodann wird im Schritt 32 in einem Gießverfahren die Negativform in der Muffel mit der Lithiumsilikat-Glasschmelze ausgefüllt. Bevorzugterweise werden Schleudergieß- oder Vakuumdruckgießverfahren benutzt, die momentan ausschließlich für den Legierungsguss insbesondere für edelmetallhaltige Legierungen oder Nichtedelmetalllegierungen auf Kobalt-Chrom oder Nickel-Chrom-Basis verwendet werden. Geeignet sind hierfür beispielsweise Anordnungen, wie sie in der DE 31 17 470 (Schleuderguss) bzw. DE 26 51 842 (Vakuumdruckguss) beschrieben sind. Hierbei berücksichtigte Parameter sind folgende:
a) Schleudergießverfahren:
   Temperatur der Schmelze: 1230 °C. Nach dem Befüllen der Muffel folgt eine Nachlaufzeit des Tiegels und der Muffel zwischen 5 min und 15 min, während der die Muffel weiterhin gedreht wird. Danach kann die Muffel entweder in einem Vorwärmofen temperiert werden (z. B. bei 600 °C) oder auf Raumtemperatur durch Stehenlassen auf einer geeigneten unbeheizten und wärmeresistenten Unterlage auf Raumtemperatur abgekühlt werden.
b) Vakuumdruckgießverfahren:
   Temperatur der Schmelze während des Gießens: 1250 °C bei einer Gießzeit von 5 min (d. h. Gusstiegel und Muffel verbleiben in dieser Zeit auf der Gussstellung). Sodann erfolgt ein Abkühlen der Muffel im Gussgerät auf 600 °C.

Vor dem Aufschmelzen der Pearls kann ein weiterer Verfahrensschritt durchgeführt werden, um beim Abschrecken aufgetretene Spannung abzubauen (Schritt 27). Hierzu können die Pearls in einem Entspannungsofen zunächst auf eine Temperatur T3 von in etwa 450 °C erwärmt und sodann stufenweise auf Raumtemperatur innerhalb einer Zeit t3 von 2 h bis 6 h abgekühlt werden.

Die Muffel mit dem den Zahnersatz bildenden erstarrten Guss kann sodann einer ersten Wärmebehandlung unterzogen werden, bei einer Temperatur T4 zwischen 600 °C und 760°C über einen Zeitraum t4 von 30 min und 120 min tritt eine Vorkristallisation ein, die Lithiummetasilikat als Hauptkristallphase aufweist (Verfahrensschritt 34). Sodann kann zur Bildung von Lithiumdisilikat als Hauptkristallphase die Muffel weiterhin in einem Vorwärmofen belassen werden, wobei eine Temperatur T6 zwischen 760°C und 860 °C über einen Zeitraum t6 von 5 min bis 20 min eingestellt wird. Hierdurch tritt die Lithiumdisilikat-Kristallbildung ein (Verfahrensschritt 36).

Alternativ kann der Guss aus der Muffel entfernt werden (Verfahrensschritt 38) und in einem Ofen dem ersten und zweiten Wärmebehandlungsschritt (Verfahrensschritte 40, 42) unterzogen werden, wie diese beschrieben worden sind. Temperatur T5 und Zeit t5 können entsprechend T4 und t4 gewählt werden.

Erfolgt die Kristallisation in der Muffel, so wird in dem Verfahrensschritt 44 der Zahnersatz entfernt und sodann im erforderlichen Umfang z. B. durch Strahlen mit Sandpartikeln nachbearbeitet (Verfahrens schritt 46). Eine entsprechende Nachbearbeitung (Schritt 48) kann auch nach dem zweiten Wärmebehandlungsschritt 42 erfolgen.

Derart hergestellter Zahnersatz weist folgende Eigenschaften auf:
Biegefestigkeit zwischen 200 MPa und 400 MPa (entsprechend DIN ISO 6872:2008). Transluzenz für sichtbares Licht von 30 - 60 % (Transmissionsmessung) bei einer Dicke von 3 mm und einer zahnähnlichen Grundfarbe (nach VITA classical shade guide ∼ A1 - A2). Säurelöslichkeit nach ISO 68772:2008 unterhalb von 100 µg/cm².

Die Herstellung der Pearls, also Tropfen, die eine kugel- oder linsenartige Geometrie aufweisen mit einer Abplattung an der unteren Seite, ist prinzipiell der Fig. 2 zu entnehmen. Mit 50 ist ein Schmelztiegel gekennzeichnet, in den die Ausgangspulvermischung eingegeben, über die Zeit t1 zwischen 4 h und 12 h bei der Temperatur T1 zwischen 1500°C bis 1600°C geschmolzen und sodann entgast wird. Die Schmelze wird einer Düse 52 zugeführt, die in Schwingung (Doppelpfeil 54) versetzt werden kann. Aus der Düse 52 tritt die Schmelze tropfenförmig (Tropfen 56) aus, die nach einer Kühlstrecke 58 auf ein Metallblech 60 fallen. Innerhalb der Kühlstrecke 58 verfestigen sich die Tropfen 56 zumindest oberflächlich in einem Umfang, dass weitgehend eine Tropfenform beim Auftreffen auf das Metallblech 52 beibehalten wird. Das Metallblech 60, das auf eine Temperatur zwischen Raumtemperatur und 500 °C eingestellt ist, bewirkt, dass die Tropfen 56 abgeschreckt werden. Von dem Metallblech 60, dessen Oberfläche zur Fallrichtung der Tropfen 48 geneigt verläuft, gelangen die Tropfen in ein Auffangbehältnis 62, das mit Wasser gefüllt sein kann oder z. B. Hochtemperaturwatte enthält. Aus dem Behältnis 62 werden sodann die als Pearls zu bezeichnenden ausgehärteten Tropfen entnommen, und ggfs. in zuvor beschriebener Weise einem Entspannungsofen zugeführt, um Spannungen abzubauen. Die Pearls werden sodann in zuvor beschriebener Weise aufgeschmolzen, um im Gießverfahren einen Zahnersatz herzustellen.

## Patentansprüche

1. Verfahren zum Herstellen von Zahnersatz auf der Basis von Lithiumsilikat-Glas oder -Glaskeramik, umfassend die Verfahrensschritte:
- Schmelzen eines Pulvers mit der Zusammensetzung in Gew.-%
| | | | |
|---|---|---|---|
| SiO2 | 50 | - | 70 % |
| Li2O | 5 | - | 25 % |
| Al2O3 | 0.1 | - | 20 % |
| K2O | 0.1 | - | 15 % |
| CeO2 | 0.1 | - | 15 % |
| B2O3 | 0 | - | 5 % |
| P2O5 | 0 | - | 15 % |
| Tb2O3 | 0 | - | 2 % |
| ZrO2 | 0 | - | 15 % |
| ZnO | 0 | - | 4 % |
zumindest ein Additiv zwischen 0,1 und 5 Gew.-% aus der Gruppe BaO, CaO, MgO, MnO, Er2O3, Gd2O3, Na20, Pr2O3, Pr6011, Sm203, TiO2, V205, Y203,
- Herstellen von aus der Schmelze erstarrten kugel- oder linsenförmigen Glaspartikeln,
- Portionieren der Glaspartikel und Einfüllen in einen Gießtiegel,
- Schmelzen der Glaspartikel in dem Gießtiegel und Einstellen auf eine Viskosität ν mit 4 dPa·s ≤ ν ≤ 80 dPa·s,
- Gießen der so hergestellten Schmelze in eine dem Zahnersatz entsprechende von einer Einbettmasse umgebene Negativform,
- Erstarrenlassen der Schmelze in der Negativform
- Kristallisieren von Lithiummetasilikat als Hauptkristallphase aus der erstarrten Schmelze durch Anwendung einer ersten Wärmebehandlung bei einer Temperatur zwischen 600 °C und 760 °C über eine Zeit zwischen 20 min und 120 min,
- Kristallisieren von Lithiumdisilikat als Hauptkristallphase in einer zweiten Wärmebehandlung bei einer Temperatur zwischen 760 °C und 860 °C über eine Zeit zwischen 5 min und 60 min
- wobei die erste und zweite Wärmebehandlung in der Einbettmasse oder nach Entfernen der erstarrten Schmelze aus der Einbettmasse erfolgt.

2. Verfahren nach Anspruch 1,
**dadurch gekennzeichnet,**
**dass** der Zahnersatz durch Schleuder- oder Vakuumdruckgießen hergestellt wird.

3. Verfahren nach Anspruch 1 oder 2,
**dadurch gekennzeichnet,**
**dass** die Schmelze mit einer Viskosität v mit 9 dPa·s bis 40 dPa·s gegossen wird.

4. Verfahren nach Anspruch 1,
**dadurch gekennzeichnet,**
**dass** die Schmelze zusätzlich zumindest ein Oxid eines Übergangsmetalls mit einer Ordnungszahl zwischen 41 und 79 wie Nb, Ta und La mit einem Gew.-%-Anteil zwischen 0 % und 8 % enthält, wobei der Gew.-%-Anteil von einem Oxid der Übergangsmetalle mit der Ordnungszahl 59, 62, 64, 68 maximal 5 % beträgt.

5. Verfahren nach zumindest Anspruch 1,
**dadurch gekennzeichnet,**
**dass** der Zahnersatz aus einer Schmelze mit der Zusammensetzung in Gew.-% hergestellt wird:
| | | | |
|---|---|---|---|
| SiO2 | 55 | - | 65 % |
| Li2O | 10 | - | 20 % |
| Al2O3 | 0,2 | - | 15% |
| K2O | 0,2 | - | 10 % |
| CeO2 | 0,1 | - | 10 % |
| B2O3 | 0 | - | 5 % |
| P2O5 | 1 | - | 10 % |
| Tb2O3 | 0 | - | 2 % |
| ZrO2 | 2 | - | 15% |
| ZnO | 0 | - | 2 % |
zumindest ein Additiv zwischen 0,1 und 5 % aus der Gruppe BaO, CaO, MgO, MnO, Er2O3, Gd2O3, Na2O, Pr2O3, Pr6O11, Sm2O3, TiO2, V2O5, und Y2O3, wobei vorzugsweise die Schmelze zusätzlich zumindest ein Oxid eines Übergangsmetalls mit einer Ordnungszahl zwischen 41 und 79 wie La, Nb und Ta mit einem Gew.-%-Anteil zwischen 0 % und 8 % enthält, wobei der Gew.-%-Anteil von einem Oxid der Übergangsmetalle mit der Ordnungszahl 59, 62, 64, 68 maximal 5 % beträgt.

6. Verfahren nach zumindest Anspruch 1,
**dadurch gekennzeichnet,**
**dass** der Zahnersatz aus einer Schmelze mit der Zusammensetzung in Gew.-% hergestellt wird:
| | | | |
|---|---|---|---|
| SiO2 | 57 | - | 63 % |
| Li2O | 11 | - | 19 % |
| Al2O3 | 0.5 | - | 10 % |
| K2O | 0.5 | - | 10 % |
| CeO2 | 0.1 | - | 10 % |
| B2O3 | 0 | - | 5 % |
| P2O5 | 1 | - | 10 % |
| Tb2O3 | 0 | - | 2 % |
| ZrO2 | 4 | - | 15 % |
| ZnO | 0 | - | 2 % |
zumindest ein Additiv zwischen 0,1 % und 5 % aus der Gruppe BaO, CaO, MgO, MnO, Er2O3, Gd2O3, Na2O, Pr2O3, Pr6O11, Sm2O3, TiO2, V2O5, und Y2O3, wobei vorzugsweise die Zusammensetzung außerdem zumindest ein Oxid eines Übergangsmetalls mit einer Ordnungszahl zwischen 41 und 79 wie La, Nb und Ta mit einem Gew.%-Anteil zwischen 0 % und 6 % enthält, wobei der Gew.-%-Anteil von einem Oxid der Übergangsmetalle mit der Ordnungszahl 59, 62, 64, 68 maximal 5 % beträgt.

7. Verfahren nach zumindest Anspruch 1,
**dadurch gekennzeichnet,**
**dass** der Zahnersatz aus einer Schmelze mit der Zusammensetzung in Gew.-% hergestellt wird:
| | | | |
|---|---|---|---|
| SiO2 | 58 | - | 62 % |
| Li2O | 13 | - | 19 % |
| Al2O3 | 1 | - | 6 % |
| K2O | 1 | - | 5 % |
| CeO2 | 0.1 | - | 5 % |
| B2O3 | 0 | - | 4 % |
| P2O5 | 2 | - | 8 % |
| Tb2O3 | 0 | - | 2 % |
| ZrO2 | 8 | - | 12 % |
| ZnO | 0 | - | 1 % |
zumindest ein Additiv zwischen 0,1 und 4 % aus der Gruppe BaO, CaO, MgO, MnO, Er203, Gd2O3, Na2O, Pr2O3, Pr6O11, Sm2O3, Ti02, V205, und Y203, wobei vorzugsweise die Zusammensetzung außerdem zumindest ein Oxid eines Übergangsmetalls mit einer Ordnungszahl zwischen 41 und 79 wie La, Nb und Ta mit einem Gew.-%-Anteil zwischen 0 % und 5 % enthält, wobei der Gew.-%-Anteil von einem Oxid der Übergangsmetalle mit der Ordnungszahl 59, 62, 64, 68 maximal 4 % beträgt.

8. Verfahren nach zumindest einem der Ansprüche 1 bis 7,
**dadurch gekennzeichnet,**
**dass** das geschmolzene Pulver mittels einer Düse vertropft wird, dass die Tropfen eine Kühlstrecke durchfallen, in der zumindest eine oberflächliche Verfestigung der Tropfen erfolgt, dass nach Durchfallen der Kühlstrecke die Tropfen nach ggfs. zuvor erfolgter Abschreckung von einer eine Flüssigkeit oder eine Hochtemperaturwatte aufweisenden Aufnahme aufgefangen werden, wobei vorzugsweise zum Abschrecken der Tropfen diese auf ein Metallelement wie Metallblech fallen, dessen Temperatur zwischen Raumtemperatur und 500 °C eingestellt wird.

9. Verfahren nach zumindest einem der vorhergehenden Ansprüche,
**dadurch gekennzeichnet,**
**dass** das Pulvergemisch über eine Zeit t1 mit 4 h ≤ t1 ≤ 12 h bei einer Temperatur T1 mit 1500 °C ≤ T1 ≤ 1600 °C geschmolzen wird Und dass vorzugsweise die Düse beim Vertropfen auf eine Temperatur T2 mit 1250 °C ≤ T2 ≤ 1450 °C eingestellt wird und/oder dass vorzugsweise die Düse beim Vertropfen mit einer Frequenz zwischen 40 Hz und 60 Hz, insbesondere 50 Hz in Schwingung versetzt wird.

10. Verfahren nach zumindest einem der vorhergehenden Ansprüche,
**dadurch gekennzeichnet,**
**dass** die aufgefangenen Tropfen über eine Zeit t3 mit 2 h ≤ t3 ≤ 6 h bei gleichzeitigem Abkühlen von einer Temperatur T3 mit 350 °C ≤ T3 ≤ 500 °C, vorzugsweise T3 ≈ 450 °C, auf Raumtemperatur zum Abbau innerer Spannungen abgekühlt werden.

11. Verfahren nach zumindest einem der vorhergehenden Ansprüche,
**dadurch gekennzeichnet,**
**dass** der Guss nach der zweiten Wärmebehandlung über eine Zeit t7 mit 10 min ≤ t7 ≤ 180 min auf Raumtemperatur abgekühlt wird.

12. Verfahren nach zumindest einem der vorhergehenden Ansprüche,
**dadurch gekennzeichnet,**
**dass** die Tropfen in einem Schmelz- oder Gießtiegel aus Graphit oder Keramik aufgeschmolzen werden, wobei bei Verwendung eines Graphittiegels dieser vorzugsweise zumindest innenseitig insbesondere mit Bornitrid beschichtet ist.

13. Verfahren nach zumindest einem der vorhergehenden Ansprüche,
**dadurch gekennzeichnet,**
**dass** die Schmelze mit einer Temperatur T8 mit 1200 °C ≤ T8 ≤ 1300 °C der Negativform zugeführt wird, wobei insbesondere die vorzugsweise gipsgebundene Einbettmasse beim Gießen der Schmelze auf eine Temperatur T9 mit 600 °C ≤ T9 ≤ 800 °C eingestellt wird.

14. Verfahren nach zumindest einem der vorhergehenden Ansprüche,
**dadurch gekennzeichnet,**
**dass** die Schmelze mit einer Viskosität ν mit 3 dPa·s ≤ ν ≤ 32 Pa·s der Düse zum Vertropfen zugeführt wird.

## Claims

1. Method for producing a dental prosthesis based on lithium silicate glass or lithium silicate glass-ceramic, comprising the method steps:
- melting a powder with the composition in wt-%
| | | | |
|---|---|---|---|
| SiO₂ | 50 | - | 70 % |
| Li₂O | 5 | - | 25 % |
| Al₂O₃ | 0.1 | - | 20 % |
| K₂O | 0.1 | - | 15 % |
| CeO₂ | 0.1 | - | 15 % |
| B₂O₃ | 0 | - | 5 % |
| P₂O₅ | 0 | - | 15 % |
| Tb₂O₃ | 0 | - | 2 % |
| ZrO₂ | 0 | - | 15 % |
| ZnO₂ | 0 | - | 4 % |
as well as at least an additive between 0.1 and 5 wt-% of an oxide from the group BaO, CaO, MgO, MnO, Er₂O₃, Gd₂O₃, Na₂O, Pr₂O₃, Pr₆O₁₁, Sm₂O₃, TiO₂, V₂O₅, Y₂O₃,
- producing spherical, lens-shaped or rod-shaped glass particles solidified from the melt;
- portioning the glass particles and filling them into a crucible;
- melting the glass particles in the crucible and setting a viscosity ν, wherein 4 dPa·s ≤ ν ≤ 80 dPa·s;
- casting the thus produced melt into a negative mold which is enclosed by an investment material and corresponds to the dental prosthesis;
- solidifying the melt in the negative mold,
- crystallizing lithium metasilicate as main crystalline phase from the solidified melt by employing a first heat treatment at a temperature between 600 °C and 760 °C over a time between 20 min and 120 min,
- crystallizing lithium disilicate as main crystalline phase during a second heat treatment at a temperature between 760 °C and 860 °C over a time between 5 min and 60 min,
- wherein the first and second heat treatment takes place within the investment material or after removal of the solidified melt from the investment material.

2. Method according to claim 1,
**characterized in that**
the dental prosthesis is produced by centrifugal casting or vacuum die-casting.

3. Method according to claim 1 or 2,
**characterized in that**
the melt is cast with a viscosity v, wherein 9 dPa·s to 40 dPa·s.

4. Method according to claim 1,
**characterized in that**
the melt additionally contains at least one oxide of a transition metal with an atomic number between 41 and 79, such as Nb, Ta, and La, with a wt-% proportion between 0 % and 8 %, wherein the wt-% proportion of an oxide of the transition metals with the atomic number 59, 62, 64, 68 is at most 5 %.

5. Method according to at least claim 1,
**characterized in that**
the dental prosthesis is used, which is produced from a melt with the composition in wt-%:
| | | | |
|---|---|---|---|
| SiO₂ | 55 | - | 65 % |
| Li₂O | 10 | - | 20 % |
| Al₂O₃ | 0.2 | - | 15 % |
| K₂O | 0.2 | - | 10 % |
| CeO₂ | 0.1 | - | 10 % |
| B₂O₃ | 0 | - | 5 % |
| P₂O₅ | 1 | - | 10 % |
| Tb₂O₃ | 0 | - | 2 % |
| ZrO₂ | 2 | - | 15 % |
| ZnO₂ | 0 | - | 2 % |
as well as at least one additive between 0.1 and 5 % from the group BaO, CaO, MgO, MnO, Er₂O₃, Gd₂O₃, Na₂O, Pr₂O₃, Pr₆O₁₁, Sm₂O₃, TiO₂, V₂O₅, and Y₂O₃, wherein preferably the melt additionally contains at least one oxide of a transition metal with an atomic number between 41 and 79, such as La, Nb, and Ta, with a wt-% proportion between 0 % and 8 %, wherein the wt-% proportion of an oxide of the transition metals with the atomic number 59, 62, 64, 68 is at most 5 %.

6. Method according to at least claim 1,
**characterized in that**
the dental prosthesis is produced from a melt with the composition in wt-%:
| | | | |
|---|---|---|---|
| SiO₂ | 57 | - | 63 % |
| Li₂O | 11 | - | 19 % |
| Al₂O₃ | 0.5 | - | 10 % |
| K₂O | 0.5 | - | 10 % |
| CeO₂ | 0.1 | - | 10 % |
| B₂O₃ | 0 | - | 5 % |
| P₂O₅ | 1 | - | 10 % |
| Tb₂O₃ | 0 | - | 2 % |
| ZrO₂ | 4 | - | 15 % |
| ZnO₂ | 0 | - | 2 % |
as well as at least one additive between 0.1 % and 5 % from the group BaO, CaO, MgO, MnO, Er₂O₃, Gd₂O₃, Na₂O, Pr₂O₃, Pr₆O₁₁, Sm₂O₃, TiO₂, V₂O₅, and Y₂O₃, wherein preferably the composition additionally contains at least one oxide of a transition metal with an atomic number between 41 and 79, such as La, Nb, and Ta, with a wt-% proportion between 0 % and 6 %, wherein the wt-% proportion of an oxide of the transition metals with the atomic number 59, 62, 64, 68 is at most 5 %.

7. Method according to at least claim 1,
**characterized in that**
the dental prosthesis is produced from a melt with the composition in wt-%:
| | | | |
|---|---|---|---|
| SiO₂ | 58 | - | 62 % |
| Li₂O | 13 | - | 19 % |
| Al₂O₃ | 1 | - | 6 % |
| K₂O | 1 | - | 5 % |
| CeO₂ | 0.1 | - | 5 % |
| B₂O₃ | 0 | - | 4 % |
| P₂O₅ | 2 | - | 8 % |
| Tb₂O₃ | 0 | - | 2 % |
| ZrO₂ | 8 | - | 12 % |
| ZnO₂ | 0 | - | 1 % |
as well as at least one additive between 0.1 and 4 % from the group BaO, CaO, MgO, MnO, Er₂O₃, Gd₂O₃, Na₂O, Pr₂O₃, Pr₆O₁₁, Sm₂O₃, TiO₂, V2O5, and Y₂O₃, wherein preferably the composition additionally contains at least one oxide of a transition metal with an atomic number between 41 and 79, such as La, Nb, and Ta, with a wt-% proportion between 0 % and 5 %, wherein the wt-% proportion of an oxide of the transition metals with the atomic number 59, 62, 64, 68 is at most 4 %.

8. Method according to at least one of the claims 1 to 7,
**characterized in that**
the melted powder is transformed into droplets by means of a nozzle, that the droplets are falling through a cooling section, in which at least a superficial solidification of the droplets takes place, that after falling through the cooling section the droplets are collected by a receptacle containing a liquid or a high temperature cotton, if necessary after quenching them beforehand, wherein preferably for quenching the droplets fall onto a metal element, such as sheet metal, the temperature of which is set between room temperature and 500 °C.

9. Method according to at least one of the preceding claims,
**characterized in that**
the powder mixture is melted over a time t1, wherein 4 h ≤ t1 ≤ 12 h at a temperature T1, wherein 1500 °C ≤ T1 ≤ 1600 °C, and that preferably during the transformation of the droplets, the nozzle is set to a temperature T2, wherein 1250 °C ≤ T2 ≤ 1450 °C, and/or that preferably during the transformation of the droplets, the nozzle is caused to vibrate at a frequency between 40 Hz and 60 Hz, particularly 50 Hz.

10. Method according to at least one of the preceding claims,
**characterized in that**
over a time t3, wherein 2 h ≤ t3 ≤ 6 h, the collected droplets are cooled down from a temperature T3, wherein 350 °C ≤ T3 ≤ 500 °C, preferably T3 ≈ 450 °C, to room temperature to reduce internal stress.

11. Method according to at least one of the preceding claims,
**characterized in that**
after the second heat treatment, the cast is cooled down to room temperature over a time t7, wherein 10 min ≤ t7 ≤ 180 min.

12. Method according to at least one of the preceding claims,
**characterized in that**
the droplets are melted in a crucible or ladle made of graphite or ceramic, wherein, when a graphite crucible is used, it is at least coated at the inside, particularly with boron nitride.

13. Method according to at least one of the preceding claims,
**characterized in that**
the melt is fed to the negative mold with a temperature T8, wherein 1200 °C ≤ T8 ≤ 1300 °C, wherein preferably the particularly gypsum-bound investment material is set to a temperature T9, when casting the melt, wherein 600 °C ≤ T9 ≤ 800 °C.

14. Method according to at least one of the preceding claims,
**characterized in that**
the melt is fed to the nozzle with a viscosity ν with 3 dPa·s ≤ ν ≤ 32 dPa·s to be transformed into droplets.

## Revendications

1. Procédé de fabrication de prothèse dentaire à base de verre de silicate de lithium ou de vitrocéramique comprenant les étapes suivantes :
- Fonte d'une poudre avec la composition en % en poids
| | | | |
|---|---|---|---|
| SiO2 | 50 | - | 70% |
| Li2O | 5 | - | 25% |
| Al2O3 | 0,1 | - | 20% |
| K2O | 0,1 | - | 15% |
| CeO2 | 0,1 | - | 15% |
| B2O3 | 0 | - | 5% |
| P2O5 | 0 | - | 15% |
| Tb2O3 | 0 | - | 2% |
| ZrO2 | 0 | - | 15% |
| ZnO | 0 | - | 4% |
au moins un additif entre 0,1 et 5 % en poids du groupe BaO, CaO, MgO, MnO, Er2O3, Gd2O3, Na2O, Pr2O3, Pr6O11, Sm2O3, TiO2, V2O5, Y2O3,
- Fabrication de particules de verre sphériques ou lenticulaires solidifiées à partir de la masse fondue,
- Mise en portion des particules de verre et remplissage dans un creuset de coulée,
- Fonte des particules de verre dans le creuset de coulée et ajustement à une viscosité v avec 4 dPa·s ≤ v ≤ 80 dPa·s,
- Coulage de la masse fondue ainsi produite dans un moule négatif correspondant à la prothèse dentaire et entouré d'une matière d'enrobage,
- Solidification de la masse fondue dans le moule négatif,
- Cristallisation du métasilicate de lithium en tant que phase cristalline principale de la masse fondue solidifiée par application d'un premier traitement thermique à une température entre 600 °C et 760 °C pour une durée comprise entre 20 min et 120 min,
- Cristallisation du disilicate de lithium en tant que phase cristalline principale au cours d'une seconde étape de traitement thermique à une température entre 760 °C et 860 °C pour une durée comprise entre 5 min et 60 min,
- Sachant que le premier et le second traitements thermiques se font dans la matière d'enrobage ou après enlèvement de la masse fondue solidifiée de la matière d'enrobage.

2. Procédé selon la revendication 1,
**caractérisé en ce**
**que** la prothèse dentaire est fabriquée par coulée centrifuge ou par coulée sous pression sous vide.

3. Procédé selon la revendication 1 ou 2,
**caractérisé en ce**
**que** la masse fondue est coulée avec une viscosité v située entre 9 dPa·s et 40 dPa·s

4. Procédé selon la revendication 1,
**caractérisé en ce**
**que** la masse fondue contient également au moins un oxyde d'un métal de transition ayant un numéro atomique entre 41 et 79 tel que Nb, Ta et La avec une quantité en poids comprise entre 0 % et 8 %, sachant que la quantité en poids d'un oxyde des métaux de transition ayant un numéro atomique 59, 62, 64, 68 est au maximum de 5 %.

5. Procédé selon au moins la revendication 1,
**caractérisé en ce**
**que** la prothèse dentaire est fabriquée à partir de la masse fondue ayant la composition en % en poids :
| | | | |
|---|---|---|---|
| SiO2 | 55 | - | 65% |
| Li2O | 10 | - | 20% |
| Al2O3 | 0,2 | - | 15% |
| K2O | 0,2 | - | 10% |
| CeO2 | 0,1 | - | 10% |
| B2O3 | 0 | - | 5% |
| P2O5 | 1 | - | 10% |
| Tb2O3 | 0 | - | 2% |
| ZrO2 | 2 | - | 15% |
| ZnO | 0 | - | 2% |
au moins un additif entre 0,1 et 5 % du groupe BaO, CaO, MgO, MnO, Er2O3, Gd2O3, Na2O, Pr2O3, Pr6O11, Sm2O3, TiO2, V2O5, et Y203, sachant que de préférence la masse fondue contient également au moins un oxyde d'un métal de transition ayant un numéro atomique entre 41 et 79 wie La, Nb et Ta avec une quantité en poids comprise entre comprise entre 0 % et 8 %, sachant que la quantité en poids d'un oxyde des métaux de transition ayant un numéro atomique 59, 62, 64, 68 est au maximum de 5 %.

6. Procédé selon au moins la revendication 1,
**caractérisé en ce**
**que** la prothèse dentaire est fabriquée à partir d'une masse fondue ayant la composition en % en poids :
| | | | |
|---|---|---|---|
| SiO2 | 57 | - | 63% |
| Li2O | 11 | - | 19% |
| Al2O3 | 0,5 | - | 10% |
| K2O | 0,5 | - | 10% |
| CeO2 | 0,1 | - | 10% |
| B2O3 | 0 | - | 5% |
| P2O5 | 1 | - | 10% |
| Tb2O3 | 0 | - | 2% |
| ZrO2 | 4 | - | 15% |
| ZnO | 0 | - | 2% |
au moins un additif entre 0,1 % et 5 % du groupe BaO, CaO, MgO, MnO, Er2O3, Gd2O3, Na2O, Pr2O3, Pr6O11, Sm2O3, TiO2, V2O5, et Y2O3, sachant que de préférence la composition contient en outre au moins un oxyde d'un métal de transition ayant un numéro atomique entre 41 et 79 tel que La, Nb et Ta avec une quantité en poids comprise entre comprise entre 0 % et 6 %, sachant que la quantité en poids d'un oxyde des métaux de transition ayant un numéro atomique 59, 62, 64, 68 est au maximum de 5 %.

7. Procédé selon au moins la revendication 1,
**caractérisé en ce**
**que** la prothèse dentaire est fabriquée à partir d'une masse fondue ayant la composition en % en poids :
| | | | |
|---|---|---|---|
| SiO2 | 58 | - | 62% |
| Li2O | 13 | - | 19% |
| Al2O3 | 1 | - | 6% |
| K2O | 1 | - | 5% |
| CeO2 | 0,1 | - | 5% |
| B2O3 | 0 | - | 4% |
| P2O5 | 2 | - | 8% |
| Tb2O3 | 0 | - | 2% |
| ZrO2 | 8 | - | 12% |
| ZnO | 0 | - | 1% |
au moins un additif entre 0,1 et 4 % du groupe BaO, CaO, MgO, MnO, Er2O3, Gd2O3, Na2O, Pr2O3, Pr6O11, Sm2O3, TiO2, V2O5, et Y2O3, sachant que la composition contient de préférence également au moins un oxyde d'un métal de transition ayant un numéro atomique entre 41 et 79 tel que La, Nb et Ta avec une quantité en poids comprise entre comprise entre 0 % et 5 %, sachant que la quantité en poids d'un oxyde des métaux de transition ayant un numéro atomique 59, 62, 64, 68 est au maximum de 4 %.

8. Procédé selon au moins l'une des revendications 1 à 7,
**caractérisé en ce**
**que** la poudre fondue s'égoutte par une buse, que les gouttes passent à travers une section de refroidissement, dans laquelle s'ensuit au moins une solidification superficielle des gouttes, qu'après avoir traversé la section de refroidissement et, après le cas échéant la trempe préalable, les gouttes sont collectées par un récipient comprenant un liquide ou de la ouate haute température, sachant que pour se refroidir lesdites gouttes tombent de préférence sur un élément métallique tel une tôle métallique, dont la température est réglée entre la température ambiante et 500 °C.

9. Procédé selon au moins l'une des précédentes revendications,
**caractérisé en ce**
**que** le mélange de poudre est fondu pendant un temps t1 avec 4 h ≤ t1 ≤ 12 h à une température T1 avec 1 500 °C ≤ T1 ≤ 1 600 °C et **en ce que**, lors de l'égouttement, la buse est de préférence réglée sur une température T2 avec 1 250 °C ≤ T2 ≤ 1 450 °C et/ou que lors de l'égouttement, de préférence la buse est soumise à des vibrations à une fréquence entre 40 Hz et 60 Hz, en particulier 50 Hz.

10. Procédé selon au moins l'une des précédentes revendications,
**caractérisé en ce**
**que** les gouttes collectées sont refroidies à température ambiante pendant un temps t3 avec 2 h ≤ t3 ≤ 6 h avec un refroidissement simultané à une température T3 avec 350 °C ≤ T3 ≤ 500 °C, de préférence T3 ≈ 450 °C afin de réduire les tensions internes

11. Procédé selon au moins l'une des précédentes revendications,
**caractérisé en ce**
**que**, après le second traitement thermique, la pièce coulée est refroidie à température ambiante pendant un temps t7 avec 10 min ≤ t7 < 180 min.

12. Procédé selon au moins l'une des précédentes revendications,
**caractérisé en ce**
**que** les gouttes sont fondues dans un creuset de fusion ou de coulée en graphite ou en céramique, sachant qu'en cas d'utilisation d'un creuset en graphite ce dernier est de préférence revêtu tout particulièrement de nitrure de bore au moins à l'intérieur.

13. Procédé selon au moins l'une des précédentes revendications,
**caractérisé en ce**
**que** la masse fondue est introduite dans le moule négatif à une température T8 avec 1 200 °C ≤ T8 ≤ 1 300 °C, sachant que lors de la coulée de la masse fondue, la masse d'enrobage, de préférence à base de plâtre, est tout particulièrement réglée à une température T9 avec 600 °C ≤ T9 ≤ 800 °C.

14. Procédé selon au moins l'une des précédentes revendications,
**caractérisé en ce**
**que** la masse fondue est amenée à la buse pour égouttement avec une viscosité v avec 3 dPa·s ≤ v ≤ 32 Pa·s.
